(19) Europäisches Patentamt
European Patent Office
Office européen des brevets

(11) Publication number: **0 069 598**
**B1**

(12) # EUROPEAN PATENT SPECIFICATION

(45) Date of publication of patent specification: **19.12.84**

(51) Int. Cl.³: **C 07 C 49/813**, C 07 C 49/83, C 07 C 49/84, C 07 C 45/45

(21) Application number: **82303569.6**

(22) Date of filing: **07.07.82**

(54) **Preparation of aromatic ketones.**

(30) Priority: **08.07.81 US 281526**

(43) Date of publication of application:
**12.01.83 Bulletin 83/02**

(45) Publication of the grant of the patent:
**19.12.84 Bulletin 84/51**

(84) Designated Contracting States:
**AT BE CH DE FR IT LI NL SE**

(56) References cited:
**DE-A-2 241 560**
**FR-A-2 167 624**
**FR-A-2 357 517**
**GB-A-1 164 046**
**GB-A-1 406 011**

(73) Proprietor: **RAYCHEM CORPORATION**
**300 Constitution Drive**
**Menlo Park California 94025 (US)**

(72) Inventor: **Dahl, Klaus J.**
**98 Juniper Drive**
**Atherton California 94025 (US)**
Inventor: **Jansons, Viktors**
**123 New York Avenue**
**Las Gatos California 95030 (US)**

(74) Representative: **Jones, David Colin et al**
**Raychem Limited Intellectual Property Law Department Swan House 37-39 High Holborn London WC1 (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 069 598 B1

**Description**

This invention relates to the preparation of diaryl ketones.

Diaryl ketones are useful in the preparation of poly(arylene ether ketones). In the preparation of these polymers it is essential that the monomers used be in a highly pure state to prevent undesirable side reactions. Furthermore, only monomers which are substituted in the para position yield a polymer having the desired properties. The present invention relates to a process for the preparation of diaryl ketones that tends to improve outstandingly the degree of purity of the product and/or the degree of para substitution.

The present invention provides a process for the preparation of an aromatic ketone of the general formula

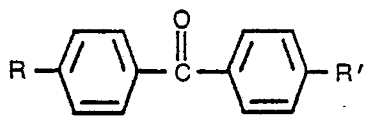 ,

wherein R and R′ may be the same or different and wherein each represents a Br, Cl, or F atom or an OH group or a group of the general formula

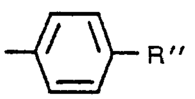 or 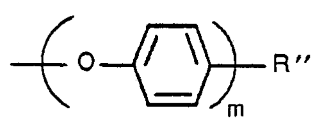

wherein m is 1 or 2 and R″ represents a Br, Cl or F atom or an OH group, which comprises reacting a substituted aromatic compound of the general formula

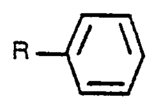

with an acyl compound of the general formula

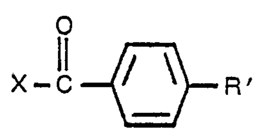 ,

wherein R and R′ are as defined above and X represents a Br, Cl or F atom or an OH or alkoxy group, in the presence of a catalyst system comprising a mixture of a Lewis acid and a strong proton acid, the Lewis acid and the strong acid each being present in an amount which is at least equimolar with the amount of the acyl compound.

Preferred examples of Lewis acids that can be used include boron trifluoride, boron trichloride, boron tribromide, titatium tetrafluoride, titanium tetrachloride, titanium tetrabromide and the pentafluorides, pentachlorides and pentabromides of tantalum, niobium, phosphorus, arsenic and antimony. Boron trifluoride and antimony pentafluoride are especially preferred.

Preferred strong acids include fluorosulphuric acid, hydrofluoric acid, perchloric acid and trifluoromethanesulphonic acid.

The process of this invention is useful in preparing precursors for poly(arylene ether ketones) since the process yields highly pure para substituted aromatic ketones with little, if any, contamination with ortho substituted isomers. Recovery of the product is described more fully below with reference to preferred embodiments of the invention. The expression "highly pure para substituted aromatic ketones" is used to specify that at least about 96%, preferably at least 98% and most preferably at least 99.5% of the aromatic ketone product consists of the para substituted isomer.

The compounds prepared in accordance with this invention are dihydric phenolic compounds, dihaloaromatic compounds and halophenols. The dihydric phenols can be reacted with the dihaloaromatic compounds or other suitable comonomers to produce the poly(arylene ether ketones). The halophenols can be polymerized by self condensation to form the poly(arylene ether ketones) or can be reacted with comonomers, such as the dihydric phenols and dihaloaromatic compounds, to form the poly(arylene ether ketones).

The substituted aromatic compound used as a starting material in the process of this invention is a mononuclear or polynuclear aromatic compound containing at least one substituent and being unsubstituted in the para position with respect to that substituent.

Typical substituted aromatic compounds which can be used as starting materials include: fluorobenzene, phenol, 4-phenoxyphenol, 4-phenylphenol, 4-fluorobiphenyl, 4-chlorobiphenyl, 4-chlorodiphenyl ether, 4-fluorodiphenyl ether, and the like. The compounds are well known, many are commercially available, and they can all be prepared by standard methods of synthesis.

The aromatic acyl compound used as a starting material is a substituted benzoic acid, or an acid halide or alkyl ester of a substituted benzoic acid and references to the alkoxy substituent represented by X are meant to include straight chain alkoxy radicals having 1—5 carbon atoms. Typical acyl compounds include 4-hydroxybenzoic acid, 4-chlorobenzoic acid, 4-fluorobenzoic acid, 4-(4-hydroxyphenoxy)benzoic acid, 4-chlorobenzoyl chloride, 4-fluorobenzoyl chloride, 4-fluorobenzoyl fluoride, ethyl 4-hydroxybenzoate, methyl 4-hydroxybenzoate, methyl 4-chlorobenzoate, ethyl 4-fluorobenzoate, methyl 4-fluorobenzoate, n-butyl 4-fluorobenzoate and the like. These acyl compounds are also well known and can be prepared by standard methods of synthesis.

Preferred acyl compounds are 4-chlorobenzoic acid, 4-chlorobenzoyl chloride or 4-chlorobenzoyl fluoride.

Preferred combinations of substituted aromatic compounds and acyl compound include a combination of fluorobenzene with 4-fluorobenzoyl chloride, 4-fluorobenzoyl fluoride, 4-fluorobenzoic acid, methyl 4-fluorobenzoate or ethyl 4-fluorobenzoate, or alternatively with 4-hydroxybenzoic acid or with 4-(4-hydroxyphenoxy)benzoic acid; a combination of phenol with 4-fluorobenzoyl chloride, 4-fluorobenzoyl fluoride, 4-fluorobenzoic acid, methyl 4-fluorobenzoate or ethyl 4-fluorobenzoate; and a combination of 4-phenoxyphenol with 4-chlorobenzoyl chloride or 4-chlorobenzoic acid or alternatively with 4-fluorobenzoyl chloride, 4-fluorobenzoyl fluoride, 4-fluorobenzoic acid methyl 4-fluorobenzoate or ethyl 4-fluorobenzoate.

In carrying out the process of this invention, equimolar amounts of the substituted aromatic compound and the aromatic acyl compound are preferably employed, although it may be advantageous in certain circumstances to use a molar excess of one reactant.

In the acid catalyst system used for the process of the invention, it is preferred to use from about 2 to about 3 moles of Lewis acid per mole of acyl reactant; and from about 2 to about 40 moles of strong acid per mole of the acyl reactant.

The process of the invention can be carried out at autogenous pressures but higher pressure can be used where desired. Usually it will be desirable to carry out the reaction under elevated pressures of about 2 to about 20 atmospheres. It is particularly preferred to conduct the reaction under conditions such that the partial pressure of the catalyst system during the course of the reaction is up to about 2 or 3 atmospheres.

The reaction medium can be a polar solvent, optionally with an inert diluent. The strong acid, particularly if hydrofluoric acid, can be used as the reaction medium, if desired. Illustrative examples of solvents that can be used are polar solvents, for example, sulphur dioxide, tetramethylene sulphone, nitrobenzene, sulphurylfluorochloride and mixtures thereof. The solvent employed is preferably such that the catalyst system forms a homogenous solution when dissolved in the solvent. To obtain a satisfactory reaction rate, it is preferable that at least one of the reactants should also form a homogeneous solution in the polar solvent and catalyst system. However, it is especially advantageous for one of the reactants (usually the substituted aromatic compound, as in Example 4) and/or the ketone product to have a solubility in the polar solvent and catalyst system of no greater than about 2.0 weight percent, particularly no greater than about 0.5 weight percent. The solvent is preferably present in an amount such that the combined weight of the reactants ranges from about 5 to about 70 weight percent of the weight of the polar solvent. Inert diluents which can be used with the polar solvent include normal alkanes containing 3 to 10 carbon atoms, and geminal polychloro-, polyfluoro- and poly(fluorochloro)-n-alkanes containing 1 to 10 carbon atoms. A preferred reaction medium comprises anhydrous hydrofluoric acid.

The reaction temperature is preferably from about −25°C to about +75°C and especially preferably in the range of about 0°C to about 35°C and particularly from about 0°C to about 20°C.

The following examples illustrate by way of example, preparation of para substituted aromatic ketones according to the process of the present invention.

Example 1

This example illustrates the preparation of 4-fluoro-4'-hydroxybenzophenone from fluorobenzene and 4-hydroxybenzoic acid.

To a mixture of 138.13 grams (1.00 mole) 4-hydroxybenzoic acid and 125.0 grams (1.30 mole) fluorobenzene, cooled in a dry ice/acetone bath, is added 300 millilitres of anhydrous hydrofluoric acid and a pre-cooled magnetic stirrer bar. The partly frozen reaction mixture is removed from the bath and stirred at −20°C to −15°C for 15 minutes yielding two liquid layers. Boron trifluoride is periodically admitted at a pressure of about 15 psi resulting in a temperature rise to 18°C in about 15 minutes. The reaction vessel is reimmersed in the dry ice/acetone bath to lower the temperature. Admission of boron trifluoride is continued at −4°C to +6°C for 40 minutes until absorption and exotherm ceases. The bath is removed and boron trifluoride is admitted at a pressure of about 30 psi. The reactor is warmed to room temperature by placing it under a lamp for about 1 hour, at which point about $\frac{1}{4}$ of the top layer is consumed. The reactor is held at room temperature until all of the top layer is consumed, about 1 hour 15 minutes. The reaction solution is stirred for an additional 18 hours and degassed. The resulting clear light yellow reaction solution is removed and evaporated to dryness at room temperature. The light orange, crusty residue is stirred with excess ice water and leached with water overnight to yield a cream colored product. The product is recrystallized from about 550 millilitres of methanol by slowly adding about 180 millilitres of water at reflux, resulting in 153.0 grams (71% yield) of colourless crystals. The melting point of the product is 170—171.5°C. The product is further purified by recrystallization from glacial acetic acid with addition of 100 millilitres of water at reflux, followed by washing the resulting filter cake with water, and recrystallizing from about 1200 ml toluene, to give 139.70 grams of colourless crystals. The melting point of the crystals is 170.6—172°C and the infrared and nuclear magnetic resonance spectra are consistent with a known sample of 4-fluoro-4'-hydroxybenzophenone. Gas-liquid chromatography shows that the product consists essentially of the para isomer.

Example 2

This example illustrates the preparation of 4-fluoro-4'-hydroxybenzophenone from phenol and 4-fluorobenzoyl chloride.

To a mixture of 110.00 g (0.694 mole) 4-fluorobenzoyl chloride and 71.00 g (0.754 mole) phenol, cooled in a dry ice/acetone bath is added 300 millilitres anhydrous hydrofluoric acid and a pre-cooled magnetic stirrer bar. The acylation reaction is carried out and worked up as in Example 1 for the reaction of 4-hydroxybenzoic acid with fluorobenzene, yielding 138.25 g (92.5% yield) of off-white crystals, having a melting point of 169.5—171.5°C on recrystallization from methanol-water. Further recrystallization from aqueous acetic acid and toluene yields 124.95 g of nearly colourless crystals, having a melting point of 169.6—172°C.

Example 3

This example illustrates the preparation of 4-chloro-4'-(4-hydroxyphenoxy)benzophenone from 4-phenoxyphenol and 4-chlorobenzoic acid.

To a mixture of 13.04 grams (0.07 mole) 4-phenoxyphenol and 10.96 grams (0.07 mole) 4-chlorobenzoic acid cooled in dry ice and acetone is added 50 millilitres of anhydrous hydrofluoric acid. The mixture is stirred in an air bath at 24°C, and degassed slowly after 10 minutes. Boron trifluoride under a pressure of 30 psi is introduced, the mixture is degassed several times and stirred for 15 hours while the pressure of boron trifluoride is maintained at 30 psi. The resulting dark solution is degassed and poured onto about 100 grams of stirred ice. The precipitated product is collected, washed with water, boiled in a mixture of methanol and water, and filtered. Recrystallization from toluene yields 15.5 grams (68.2%) of a pink crystalline product. This product is recrystallized twice from an acetic acid/water mixture to yield 10.7 grams of off-white crystals. The melting point of the product is 141—142°C, and the infrared and nuclear magnetic resonance spectra are consistent with a known sample of 4-chloro-4'-(4-hydroxyphenoxy)benzophenone. Gas-liquid chromatography shows that the product consists essentially of the para isomer. No evidence of contamination with ortho or meta isomers is observed.

Example 4

This example illustrates the preparation of 4,4'-difluorobenzophenone from fluorobenzene and 4-fluorobenzoyl chloride.

To a mixture of 100.0 grams (1.04 moles) of fluorobenzene and 158.6 grams (1.00 moles) of 4-fluorobenzoyl chloride, pre-cooled to about −20°C is added 550 millilitres of cold (−10°C) anhydrous hydrofluoric acid. The resulting suspension is slowly agitated while admitting boron trifluoride gas. The reaction is allowed to rise to a temperature of about +5°C and the addition of boron trifluoride is then stopped. The resulting two phase liquid mixture is agitated for about 0.5 hours and then vented to expel hydrogen chloride. Addition of boron trifluoride is resumed and the pressure of the system permitted to rise to about 30 psi while the reaction temperature increases to about 20°C. Agitation is continued for 10 hours at room temperature to cause the virtual disappearance of the upper (fluorobenzene) layer.

The yellow reaction mixture is evaporated to dryness at room temperature. The crystalline residue is washed with water, filtered, and recrystallized from aqueous methanol to yield 180 grams (0.89 moles) of 4,4′-difluorobenzophenone. The melting point of the product is 104—107°C. Recrystallization from heptane gives off-white crystals having a melting point of 105—107°C. Infrared and nuclear magnetic resonance spectra of this material are identical to those recorded for authentic 4,4′-difluorobenzophenone. Gas-liquid chromatography shows that the product consists essentially of the para isomer.

Example 5

This example illustrates the preparation of 4,4′-dihydroxybenzophenone from phenol and 4-hydroxybenzoic acid.

To a mixture of 94.00 grams (1.00 mole) phenol and 138.0 grams (1.00 mole) 4-hydroxybenzoic acid, cooled in a dry ice/acetone bath is added 400 millilitres anhydrous hydrofluoric acid and a magnetic stirrer bar. The acylation reaction is carried out and worked up as in Example 1. A light pink crystalline crude product is obtained. Recrystallization from water containing 10% ethanol gives 180.2 g (84% yield) of nearly colourless crystals. The melting point of the product is 210—213°C and the infrared and nuclear magnetic resonance spectra are identical to those of authentic 4,4′-dihydroxybenzophenone. Gas-liquid chromatography shows that the product consists essentially of the para isomer.

Example 6

This example illustrates the preparation of poly(benzophenone ether) from 4-fluoro-4′-hydroxybenzophenone obtained by the process described in Example 1.

A solution of 1.0010 grams (4.740 millimoles) 4-fluoro-4′-hydroxybenzophenone in 4.36 millilitres (4.36 millimoles) 1.000 Normal potassium hydroxide (in 90% methanol) is evaporated to dryness in a nitrogen stream at 100—120°C. The residue is dried 30 minutes at 120—160°C under vacuum. The resulting bright yellow crystalline powder is mixed with 0.400 gram of diphenyl sulphone and the mixture is heated under an atmosphere of nitrogen for 9 minutes at about 360—366°C with occasional stirring. The hot viscous melt is spread on the walls of the reactor and cooled at room temperature. The resulting solidified film is cut into small pieces, boiled with acetone, water and methanol, and dried at 120°C at a pressure of 0.1 mm Hg, to give a light ivory colored polymer having an inherent viscosity of 1.57 (0.1 gram/100 millilitres of concentrated sulphuric acid at 25°C). The inherent viscosity is determined according to the method of Sorenson et al., Preparative Methods of Polymer Chemistry, Interscience (1968), page 44. The product is compression moulded under a pressure of 10,000 psi at a temperature of 400°C for 2 minutes to give a tough and flexible light colored slab.

**Claims**

1. A process for the preparation of an aromatic ketone of the general formula

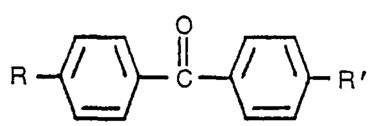 ,

wherein each of R and R′ may be the same or different, and wherein each represents a Br, Cl or F atom or an OH group, or a group of the general formula.

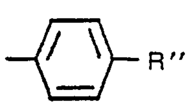 or 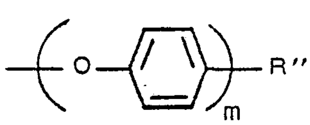

wherein m is 1 or 2 and R″ represents a Br, Cl or F atom or an OH group, which comprises reacting a substituted aromatic compound of the general formula

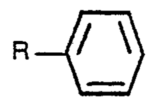

with and an acyl compound of the general formula

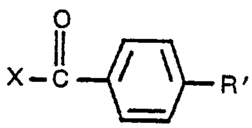

wherein R and R' are as defined above and X is a Br, Cl or F atom or an OH or alkoxy group, in the presence of a catalyst system comprising a mixture of a Lewis acid which comprises boron trifluoride, boron trichloride, boron tribromide, titanium tetrafluoride, titanium tetrachloride, titanium tetrabromide or a pentafluoride, pentachloride or pentabromide of tantalum, niobium, phosphorus, arsenic or antimony, and a strong acid the Lewis acid and the strong acid each being present in an amount which is at least equimolar with the amount of the acyl compound.

2. A process according to Claim 1, wherein the strong acid comprises fluorosulphuric acid, hydrofluoric acid, perchloric acid or trifluoromethanesulphonic acid.

3. A process according to any preceding claim, wherein equimolar amounts of said aromatic compound and said acyl compound are reacted together.

4. A process in accordance with any preceding claim wherein said reaction is carried out in a solvent comprising sulphur dioxide, tetramethylene sulphone, nitrobenzene, sulphurylfluorochloride or a mixture of two or more of these.

5. A process in accordance with Claim 4, wherein said solvent contains an inert diluent comprising a $C_3$ to $C_{10}$ n-alkane, or a $C_1$ to $C_{10}$ geminal polychloro-, polyfluoro- or poly(fluorochloro)-n-alkane.

6. A process in accordance with Claim 4 or 5, wherein the combined weight of said substituted aromatic compound, said catalyst system and said acyl compound together ranges from about 5 up to about 70 weight percent of the weight of said solvent.

7. A process in accordance with any one of Claims 4 to 6 wherein said substituted aromatic compound, said catalyst system and said acyl compound form a homogeneous solution in said solvent.

8. A process in accordance with any one of Claims 1 to 3, wherein said reaction is carried out in a reaction medium comprising anhydrous hydrofluoric acid.

9. A process in accordance with any preceding claim, wherein at least one of said substituted aromatic compound, said acyl compound and said aromatic ketone have a solubility in said catalyst system of no greater than about 2.0 weight percent.

10. A process in accordance with any one of Claims 4 to 7, wherein at least one of said substituted aromatic compound, said acyl compound and said aromatic ketone have a solubility in said solvent and catalyst system of no greater than about 2.0 weight percent.

11. A process in accordance with any preceding claim, wherein said reaction is effected at a temperature ranging from about −25°C to about +75°C.

12. A process in accordance with any preceding claim, wherein said reaction is effected at a temperature ranging from about 0°C to about +35°C.

13. A process in accordance with any preceding claim, wherein said reaction is effected at a temperature ranging from about 0°C to about +20°C.

14. A process in accordance with any preceding claim, wherein the partial pressure of said catalyst system during the course of said reaction is up to about 3 atmospheres.

15. A process in accordance with any preceding claim, wherein said acyl compound comprises 4-chlorobenzoic acid, 4-chlorobenzoyl chloride, 4-chlorobenzoyl fluoride, 4-fluorobenzoyl chloride, 4-fluorobenzoyl fluoride, 4-fluorobenzoic acid, methyl 4-fluorobenzoate, ethyl 4-fluorobenzoate, 4-hydroxybenzoic acid or 4-(4-hydroxyphenoxy)benzoic acid.

16. A process in accordance with any preceding claim, wherein said substituted aromatic compound comprises fluorobenzene, phenol or 4-phenoxyphenol.

**Revendications**

1. Procédé de préparation d'une cétone aromatique de formule générale:

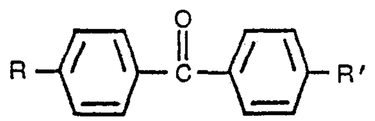

,

où chacun des radicaux R et R' peut être semblable ou différent, et où chacun d'entre eux représente un atome de Br, Cl ou F ou un groupe OH, ou un groupe de formule générale

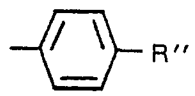

ou

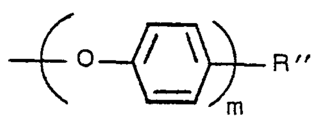

où m vaut 1 ou 2 et R'' représente un atome de Br, Cl ou F ou un groupe OH, dans lequel on fait réagir un composé aromatique substitué de formule générale:

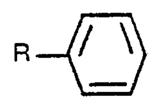

avec un composé acyle de formule générale:

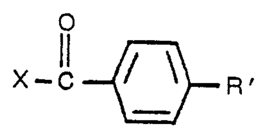

,

où R et R' sont tels que définis ci-dessus et X est un atome de Br, Cl ou F ou un groupe OH ou alcoxy, en présence d'un système catalyseur comprenant un mélange d'un acide de Lewis qui comprend le trifluorure de bore, le trichlorure de bore, le tribromure de bore, le tétrafluorure de titane, le tétrachlorure de titane, le tétrabromure de titane ou un pentafluorure, pentachlorure ou pentabromure de tantale, de niobium, de phosphore, d'arsenic ou d'antimoine, et d'un acide fort, l'acide de Lewis et l'acide fort étant l'un et l'autre présents en une quantité qui est au moins équimolaire avec la quantité du composé acyle.

2. Procédé selon la revendication 1, où l'acide fort comprend l'acide fluorosulfurique, l'acide fluorhydrique, l'acide perchlorique ou l'acide trifluorométhanesulfonique.

3. Procédé selon l'une quelconque des revendications précédentes, où l'on fait réagir ensemble des quantités équimolaires dudit composé aromatique et dudit composé acyle.

4. Procédé selon l'une quelconque des revendications précédentes, où ladite réaction est conduite dans un solvant comprenant le dioxyde de soufre, la tétraméthylènesulfone, le nitrobenzène, le fluorochlorure de sulfuryle ou un mélange de deux ou plusieurs de ces corps.

5. Procédé selon la revendication 4, où ledit solvant contient un diluant inerte comprenant un n-alcane en $C_3$ à $C_{10}$, ou un polychloro-, polyfluoro- ou poly(fluorochloro)-n-alcane géminé en $C_1$ à $C_{10}$.

6. Procédé selon l'une des revendications 4 et 5, où le poids combiné dudit composé aromatique substitué, dudit système catalyseur et dudit composé acyle ensemble va d'environ 5 à environ 70% en poids du poids dudit solvant.

7. Procédé selon l'une quelconque des revendications 4 à 6, où ledit composé aromatique substitué, ledit système catalyseur et ledit composé acyle forment une solution homogène dans ledit solvant.

8. Procédé selon l'une quelconque des revendications 1 à 3, où ladite réaction est conduite dans un milieu réactionnel comprenant de l'acide fluorhydrique anhydre.

9. Procédé selon l'une quelconque des revendications précédentes, où au moins un corps parmi ledit composé aromatique substitué, ledit composé acyle et ladite cétone aromatique a une solubilité dans ledit système catalyseur ne dépassant pas environ 2,0% en poids.

10. Procédé selon l'une quelconque des revendications 4 à 7, où au moins un corps parmi ledit composé aromatique substitué, ledit composé acyle et ladite cétone aromatique a une solubilité dans ledit solvant et ledit système catalyseur ne dépassant environ 2,0% en poids.

11. Procédé selon l'une quelconque des revendications précédentes, où ladite réaction est conduite à une température allant d'environ −25°C à environ +75°C.

12. Procédé selon l'une quelconque des revendications précédentes, où ladite réaction est conduite à une température allant d'environ 0°C à environ +35°C.

13. Procédé selon l'une quelconque des revendications précédentes, où ladite réaction est conduite à une température allant d'environ 0°C à environ +20°C.

14. Procédé selon l'une quelconque des revendications précédentes, où la pression partielle dudit système catalyseur au cours de ladite réaction va jusqu'à environ 3 atmosphères.

15. Procédé selon l'une quelconque des revendications précédentes, où ledit composé acyle comprend l'acide 4-chlorobenzoïque, le chlorure de 4-chlorobenzoyle, le fluorure de 4-chlorobenzoyle, le chlorure de 4-fluorobenzoyle, le fluorure de 4-fluorobenzoyle, l'acide 4-fluorobenzoïque, le 4-fluorobenzoate de méthyle, le 4-fluorobenzoate d'éthyle, l'acide 4-hydroxybenzoïque ou l'acide 4-(4-hydroxyphénoxy)-benzoïque.

16. Procédé selon l'une quelconque des revendications précédentes, où ledit composé aromatique substitué comprend le fluorobenzène, le phénol ou le 4-phénoxyphénol.

**Patentansprüche**

1. Verfahren zur Herstellung eines aromatischen Ketons der allgemeinen Formel

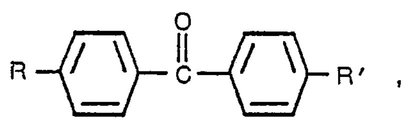

worin R und R′ gleich oder ungleich sein können und jeder ein Br-, Cl- oder F-Atom oder eine OH-Gruppe oder eine Gruppe der allgemeinen Formel

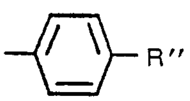

oder

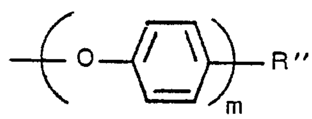

darstellt, worin m 1 oder 2 ist und R″ ein Br-, Cl- oder F-Atom oder eine OH-Gruppe darstellt, bei welchem eine substituierte aromatische Verbindung der allgemeinen Formel

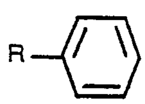

mit einer Acylverbindung der allgemeinen Formel

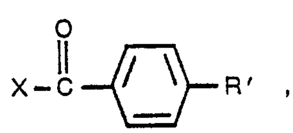

umgesetzt wird, worin R und R′ wie oben definiert sind und X ein Br-, Cl- oder F-Atom oder ein OH-Gruppe oder eine Alkoxygruppe ist, in Gegenwart eines Katalysatorsystems, das ein Gemisch einer Lewissäure, umfassend Bortrifluorid, Bortrichlorid, Bortribromid, Titantetrafluorid, Titantetrachlorid, Titantetrabromid oder ein Pentafluorid, Pentachlorid oder Pentabromid von Tantal, Niob, Phosphor, Arsen oder Antimon, und einer starken Säure ist, und die Lewissäure und die starke Säure jeweils in einer Menge anwesend sind, die mindestens äquimolar mit der Menge an Acylverbindung ist.

2. Verfahren nach Anspruch 1, in welchem die starke Säure Fluorschwefelsäure, Fluorwasserstoffsäure, Perchlorsäure oder Trifluormethansulfonsäure ist.

3. Verfahren nach einem der vorhergehenden Ansprüche, bei welchem äquimolare Mengen der aromatischen Verbindung und der Acylverbindung miteinander umgesetzt werden.

4. Verfahren nach einem der vorhergehenden Ansprüche, bei welchem die Reaktion in einem Lösungsmittel durchgeführt wird, das Schwefeldioxid, Tetramethylensulfon, Nitrobenzol, Sulfurylfluorchlorid oder ein Gemisch von zwei oder mehr derselben enthält.

5. Verfahren nach Anspruch 4, bei welchem das Lösungsmittel ein inertes Verdünnungsmittel, umfassend ein $C_3$- bis $C_{10}$-N-Alkan oder ein $C_1$- bis $C_{10}$-geminales Polychlor-, Polyfluor- oder Poly(fluorchlor)-n-alkan, enthält.

6. Verfahren nach Anspruch 4 oder 5, bei welchem das kombinierte Gewicht von substituierter aromatischer Verbindung, Katalysatorsystem und Acylverbindung zusammen etwa 5 bis etwa 70 Gewichtsprozent vom Gewicht des Lösungsmittels beträgt.

7. Verfahren nach einem der Ansprüche 4 bis 6, bei welchem die substituierte aromatische Verbindung, das Katalysatorsystem und die Acylverbindung eine homogene Lösung im Lösungsmittel bilden.

8. Verfahren nach einem der Ansprüche 1 bis 3, bei welchem die Reaktion in einem Reaktionsmedium durchgeführt wird, das wasserfreie Fluorwasserstoffsäure enthält.

9. Verfahren nach einem der vorhergehenden Ansprüche, bei welchem mindestens eine der substituierten aromatischen Verbindung, der Acylverbindung und des aromatischen Ketons eine Löslichkeit im Katalysatorsystem von nicht mehr als etwa 2,0 Gewichtsprozent hat.

10. Verfahren nach einem der Ansprüche 4 bis 7, bei welchem mindestens eine der substituierten aromatischen Verbindung, der Acylverbindung und des aromatischen Ketons eine Löslichkeit im Lösungsmittel und Katalysatorsystem von nicht mehr als etwa 2,0 Gewichtsprozent hat.

11. Verfahren nach einem der vorhergehenden Ansprüche, bei welchem die Reaktion in einem Temperaturbereich von etwa −25°C bis etwa +75°C durchgeführt wird.

12. Verfahren nach einem der vorhergehenden Ansprüche, bei welchem die Reaktion in einem Temperaturbereich von etwa 0°C bis etwa +35°C durchgeführt wird.

13. Verfahren nach einem der vorhergehenden Ansprüche, bei welchem die Reaktion in einem Temperaturbereich von etwa 0°C bis etwa +20°C durchgeführt wird.

14. Verfahren nach einem der vorhergehenden Ansprüche, bei welchem der Partialdruck des Katalysatorsystems im Verlauf der Reaktion bis zu etwa 3 Atmosphären beträgt.

15. Verfahren nach einem der vorhergehenden Ansprüche, bei welchem die Acylverbindung 4-Chlorbenzoesäure, 4-Chlorbenzoylchlorid, 4-Chlorbenzoylfluorid, 4-Fluorbenzoylchlorid, 4-Fluorbenzoylfluorid, 4-Fluorbenzoesäure, Methyl-4-fluorbenzoat, Ethyl-4-fluorbenzoat, 4-Hydroxybenzoesäure oder 4-(4-Hydroxyphenoxy)benzoesäure umfasst.

16. Verfahren nach einem der vorhergehenden Ansprüche, bei welchem die substituierte aromatische Verbindung Fluorbenzol, Phenol oder 4-Phenoxyphenol umfasst.